# EUROPEAN PATENT APPLICATION

(11) **EP 4 729 003 A1**
(43) Date of publication of application: **22.04.2026**
(21) Application number: 24207269.2
(22) Date of filing: 17.10.2024
(51) Int. Cl.: A61B 17/32

(54) **ULTRASONIC SURGICAL TOOL AND ULTRASONIC SURGICAL BLADE**

(71) Applicant: Bosonic AG, 3010 Bern (CH)
(72) Inventor: BÜSCHER, Robin, Heikendorf (DE); VELTMEIJER, Ewoud, Bern (CH); BELAL, Rafik, Bern (CH)
(74) Representative: Frei Patent Attorneys

(57) **Abstract**

An ultrasonic surgical tool (100) comprising a handpiece (20) and an ultrasonic surgical blade (10), wherein the handpiece (20) comprises a receptacle (22) for the ultrasonic surgical blade (10), the receptacle (22) being configured for transmitting ultrasonic vibrations from an ultrasonic transducer to the ultrasonic surgical blade (10) when the ultrasonic surgical blade (10) is operatively coupled to the receptacle (22), wherein the ultrasonic surgical blade (10) comprises a generally plane blade body (11) with a slot (1), wherein the plane body (11) extends, in a longitudinal direction of the ultrasonic surgical blade (10), between a proximal connection end (13) and a distal tip end (14) and comprises a proximally located fastening portion (12a) and a distally located working portion (12b), and wherein the slot (1) extends from the connection end (13) towards the tip end (14) so as to form the fastening portion (12a) of the blade body (11) with two proximally oriented prongs (15, 16).

## Description

### FIELD OF THE INVENTION

The present invention relates to the field of ultrasonic instruments. It relates to an ultrasonic surgical tool, in particular for cutting or abrasive machining, and to an ultrasonic surgical blade.

### BACKGROUND OF THE INVENTION

Ultrasonic instruments have a generator of ultrasonic energy and a surgical end effector and are used for the safe and effective treatment of many medical conditions. Ultrasonic vibration is induced in the surgical end effector by electrically exciting a transducer, for example. The transducer may be constructed of one or more piezoelectric or magnetostrictive elements in the instrument hand piece. Vibrations generated by the transducer are transmitted to the proximal end of the surgical end effector either directly or via an ultrasonic waveguide extending from the transducer to the surgical end effector. The end effector and optionally the waveguide is designed to resonate at the same frequency as the transducer. Therefore, when an end effector is attached to a transducer the overall system frequency is the same frequency as the transducer itself.

Ultrasonic vibrations, when transmitted to organic tissue at suitable energy levels and using a suitable end effector, may be used to cut, dissect, abrade, elevate or cauterize tissue or to separate muscle tissue off bone. Thus, ultrasonic surgical tools may advantageously be used to cut, dissect, abrade and/or coagulate organic tissue using energy in the form of mechanical vibrations transmitted to the surgical end effector at ultrasonic frequencies.

As ultrasonic vibrations cause dynamic friction, a tight fit is required between the end effector and the transducer or waveguide in order to achieve an efficient and effective excitation of the end effector. For this reason, ultrasonic surgical tools typically involve threads with which the end effector can be firmly connected to the transducer or waveguide.

US5695510 shows attachment of a blade by means of a bayonet joint with threads.

However, this type of attachment has some disadvantages. For example, end effectors with threads are generally manufactured by elaborate or time-consuming processes such as machining or sintering, which makes them relatively expensive. They are also relatively cumbersome and laborious to install. In any case, they cannot be replaced or swapped quickly during surgery.

There is a need for ultrasonic surgical tools adapted for ultrasonic surgical blades of simple construction that can be manufactured efficiently and economically.

Moreover, it is known to provide ultrasonic surgical tools with conduits for cooling fluid. The fluid can be water or a mixture of water with ethanol and/or a disinfectant fluid. The fluid is used to cool down the blade and to rinse away the cut material. Conduits can be double walled in order to provide for an additional conduit for aspiring fluid material, as shown in US 4515583 and US 6165150 A. Furthermore, it is known to have cooling water channels that branch out to a plurality if exit openings, as in US 5188102 or to be made of a porous, sintered material to allow water to exit through the surface of an ultrasonic cutting blade, as in US 2015/0005774 A1.

However, to have an efficient cooling of an ultrasonic surgical blade during bone cutting is still a challenge today.

### SUMMARY OF THE INVENTION

It is therefore an object of the invention to provide an ultrasonic surgical tool which overcomes or at least mitigates at least some of the disadvantages mentioned above. A further object is to provide an ultrasonic surgical blade which can be manufactured with simple means and at low cost.

These objects are solved by an ultrasonic surgical instrument and an ultrasonic surgical blade as defined in the claims. Further objects and various advantages emerge from the description and embodiments below.

The object is solved in particular by an ultrasonic surgical tool comprising a handpiece and an ultrasonic surgical blade. The handpiece comprises a receptacle for the ultrasonic surgical blade, wherein the receptacle is configured for transmitting ultrasonic vibrations from an ultrasonic transducer to the ultrasonic surgical blade when the ultrasonic surgical blade is operatively coupled to the receptacle. The ultrasonic surgical blade comprises a generally plane blade body with a slot. The plane body extends, in a longitudinal direction of the ultrasonic surgical blade, between a proximal connection end and a distal tip end and comprises a proximally located fastening portion and a distally located working portion. The slot extends from the connection end towards the tip end so as to form the fastening portion of the generally plane blade body with two proximally oriented prongs.

Such an ultrasonic surgical tool has several advantages. One advantage is that a generally plane, i.e. planar, blade body can be inexpensively manufactured out of a sheet metal blank, for example by one of, or a combination of, stamping, laser cutting, plasma cutting, waterjet cutting, chemical etching, and forging. In contrast, the ultrasonic cutting blades known from the prior art, which have a thread for attachment to a handpiece and therefore do not feature a "generally plane" blade body in the sense of the present invention, require more laborious and more expensive manufacturing methods. The aforementioned manufacturing operations can also involve creating structures on the surface of the blade body, such as teeth for cutting and abrasive operations. Yet another advantage, which is due to the two proximally oriented prongs, is that the blade can be inserted into the receptacle in a guided manner. To this end, the receptacle may comprise a guide lug or lateral limiting surfaces for the prongs. This effectively prevents the blade from shifting in the receptacle and/or falling out of the receptacle, as will be described in further detail below.

In the context of the invention, the terms "generally plane" means what is usually understood as flat by the person skilled in the art. For example, the known ultrasonic surgical blades comprising a thread or bayonet coupling at the proximal connecting end do not feature a generally plane blade body. In contrast, in the context of the invention, the blade body, i.e. both the fastening portion and the working portion of the blade body, are generally plane. In particular, the term "generally plane" means that a thickness measured as a distance between a top plane surface of the blade body and a bottom plane surface of the blade body opposite the top plane surface varies by at most 100%, preferably by at most 80%, more preferably by at most 50%, even more preferably by at most 20%, when viewed in the longitudinal direction of the blade body, relative to an average thickness value of the blade body.

In embodiments, the blade body comprises at least two slots extending from the connection end towards the tip end so as to form the fastening portion of the blade body with at least three proximally oriented prongs.

In embodiments, the blade body comprises exactly one slot, i.e. a single slot. Preferably, said exactly one slot runs in and along a longitudinal centre line L of the blade body.

In embodiments, the slot is u-shaped or v-shaped, with a width of the slot increasing in the direction of the proximal connection end. Such a design helps to align the ultrasonic surgical blade with the receptacle and the user does not need to worry about the ultrasonic surgical blade shifting out of this alignment. If necessary, the connection between the ultrasonic surgical blade and the receptacle may be further tightened, as will be described further below, to make the ultrasonic surgical tool ready for use.

In embodiments, the slot is dimensioned such that a part of the slot extends beyond the receptacle when the ultrasonic surgical blade and the receptacle are operatively coupled. This brings the advantage that a cooling liquid and/or an irrigation fluid can be supplied from the inside of the receptacle or handpiece via the slot and onto the blade. This provides for an effective cooling of the blade without large jets of water in the working area, which can improve visibility in the working area.

In embodiments, the receptacle comprises an opening with an open front end, a back end opposite the front end, a first engagement surface arranged between the front end and the back end, and a second engagement surface opposite the first engagement surface and arranged between the front end and the back end. The first and second engagement surfaces define a coupling slot therebetween. The coupling slot is configured to receive the ultrasonic surgical blade. The first and second engagement surfaces ensure that the ultrasonic surgical blade is always placed in a defined, reproducible position within the receptacle. They make it easier to insert and align the blade as they provide a clear guide for the user. This saves time when changing ultrasonic surgical blades and reduces the likelihood of errors.

The back end may define a locating surface, also known as a stop surface. In other words, at least one of the proximally oriented prongs abuts the back end of the opening when the ultrasonic surgical blade and the receptacle are operatively coupled. The locating surface helps to prevent insertion of the ultrasonic surgical blade at different depths or at an angle in the receptacle. The locating surface prevents this by providing a clear boundary for the blade so that it sits correctly. Also, the contact between the locating surface and the blade stabilizes the position of the ultrasonic surgical blade. In other words, the locating surface assists the user in positioning the blade correctly in the receptacle and helps to reduce unwanted vibrations and movement during operation, resulting in an improved ultrasonic surgical blade performance.

In embodiments, the coupling slot has at least one of a height hₛ and a width wₛ dimensioned so as to frictionally engage the ultrasonic surgical blade received in the coupling slot. The height h of the coupling slot is measured as a distance between the first and the second engagement surface. The width w of the coupling slot is measured transversely to the height h. The term "frictional engagement" refers to a force-fit or frictionally engaged connection between the respective surfaces of the ultrasonic surgical blade and the receptacle that are in contact with each other. Frictional engagement of the ultrasonic surgical blade and the coupling slot reduces the risk of the ultrasonic cutting tool slipping out of the receptacle or becoming loose during operation, which could lead to tool damage or personal injury.

In embodiments, at least one of said proximally oriented prongs comprises at least one fastening element engageable with at least one retention element of the receptacle complemental to the fastening element. The at least one fastening element and the at least one retention element are configured for detachably retaining the ultrasonic surgical blade on the handpiece with the fastening element and the retention element in cooperation. Such a detachable connection provides excellent resistance to vibrations and shocks and is particularly suitable for applications where components are subjected to motion or impact, such as during surgery.

Preferably both of said proximally oriented prongs comprise at least one fastening element engageable with at least one retention element of the receptacle complemental to the fastening elements.

In embodiments, the two proximally oriented prongs are flexible, in particular in a direction of extension of the blade body and relative to one another. In other words, the two prongs can be pressed together, which makes it particularly easy to insert the ultrasonic surgical blade into the receptacle. After the prongs have been pressed together and inserted into the open front end of the receptacle, the ultrasonic surgical blade can be inserted into its working position in which the prongs can either provide a frictionally engaged connection to the receptacle simply by means of the preload force. Alternatively or additionally, if at least one of the prongs has at least one fastening element, these fastening elements may engage in the desired position with at least one complementary retention element of the receptacle in order to provide a form-fitting connection to the receptacle. In both cases, this prevents the ultrasonic surgical blade from falling out of the receptacle even before the ultrasonic surgical blade may be securely tightened on the receptacle or handpiece with an additional connecting member.

In the context of the invention, the term "flexible" means capable of bending, deforming, or adjusting shape under applied stress or force without breaking, cracking, or permanently deforming. In particular, the term "flexible" means that the material from which the ultrasonic surgical blade is made has a modulus of elasticity (Young's modulus) between 30 GPa and 300 GPa, preferably between 100 GPa and 220 GPa, determined according to DIN EN ISO 6892-1.

In embodiments, the blade body comprises a top plane surface, a bottom plane surface, and a blade thickness d_{b}. The blade thickness d is measured as a distance between the top plane surface and the bottom plane surface, i.e. excluding any chamfers or cutting edges. The blade thickness d_{b} is between 0.1 mm and 1 mm, preferably between 0.4 mm and 0.8 mm. A blade thickness within the specified range both ensures that the blade is suitable for use with ultrasonic vibrations and sets it apart from the known ultrasonic surgical blades with threads. Alternatively or additionally, the blade body is generally plane over at least 80% of the working portion of the blade body, i.e. the blade thickness d_{b} is essentially constant over at least 80% of the working portion of the blade body.

In embodiments, each of said proximally oriented prongs has a free end, an inside edge facing the slot, an outside edge facing away from the slot, a top surface and a bottom surface opposite the top surface. Said at least one fastening element is selected from the group consisting of
- a protrusion extending laterally outwardly on its outside edge;
- a notch on its outside edge;
- a protrusion extending laterally inwardly on its inside edge;
- a notch on its inside edge;
- a free end tapering in the direction of the free end, in particular a free end tapering in the direction of the free end and towards the slot (in other words, the free end of one or both proximally oriented prongs tapers from the respective outside edge to the respective inside edge);
- a taper of at least one of said proximally oriented prongs away from the free end and towards the distal tip end;
- a geometric feature, in particular a corrugation, for increasing friction of the fastening portion with the receptacle.

In the context of the invention, the term "laterally outwardly" means a direction away from the longitudinal centre line of the blade body, in particular away from the respective inside edges of the two proximally oriented prongs.

In the context of the invention, the term "laterally inwardly" means a direction towards the longitudinal centre line of the blade body.

In embodiments, the proximally oriented prongs are connected to each other by at least one bridging element. The bridging element spans the slot and is monolithic with the blade body. It was surprisingly found that the presence of at least one bridging element connecting the proximally oriented prongs significantly improves the mechanical stability of the ultrasonic surgical blade as well as its ultrasonic excitability while still allowing the proximally oriented prongs to flex, which has the advantages described above in terms of mounting the ultrasonic surgical blade in the receptacle. Of course, in embodiments comprising at least one bridging element, the bridging element or, in the case of a plurality of bridging elements, the most proximally arranged bridging element spans the slot such that a proximal slot is still present. In particular, the at least one bridging element or, in the case of a plurality of bridging elements, the most proximally arranged bridging element is arranged such that the proximal slot still amounts to at least 10%, relative to a total length of the slot.

In embodiments where the proximally oriented prongs are connected to each other by at least one bridging element spanning the slot, the bridging elements, i.e. all bridging elements combined, extend over at most 30%, preferably at most 15%, more preferably at most 5%, of the total length of the slot. This leaves enough slot-length for cooling.

As is usually implied by the term "monolithic", this means that the blade body and the bridging element are shaped as a single piece.

The shape of the ultrasonic surgical blade can define one or more important aspects of the ultrasonic surgical tool, such as the visibility of the blade and its relative position in the surgical field, the ability of the blade to access or approach targeted tissue, the manner in which ultrasonic energy is coupled to tissue for cutting and coagulation, and/or the manner in which tissue can be manipulated with the ultrasonically inactive blade.

In embodiments, the working portion of the blade body comprises at least one cutting edge. Preferably, the blade body comprises the at least one cutting edge in the working portion only. In other words, the blade body is free of cutting edges except for in the working portion of the blade body. This makes the ultrasonic surgical blade as a whole cheaper to produce and also makes the ultrasonic surgical blade safer to use, because the user has portions of the blade body, in particular the fastening portion, which have no sharpened cutting edges and can therefore be held and handled without risking injuries. By limiting the cutting edges to the working portion, the receptacle that is in contact with the fastening portion is also less prone to being damaged by the ultrasonic cutting blade.

It is conceivable that the working portion of the blade body has cutting edges on all three edges, i.e. at the distal tip end as well as on the lateral side edges adjacent to the distal tip end. Such an ultrasonic surgical blade can be used by the surgeon in all positions and is therefore highly effective. However, it is also conceivable that the working portion of the blade body does not have cutting edges on all three edges. For example, only the distal tip end may have a cutting edge, or the distal tip end and one of the lateral side edges of the blade body. Such embodiments allow greater control and protection of tissue towards the edges that are devoid of cutting edges.

In embodiments where the working portion of the blade body comprises at least one cutting edge, the blade body has a generally rectangular shape defined by a pair of opposing lateral cutting edges and a straight distal cutting edge.

In embodiments where the working portion of the blade comprises at least one cutting edge, the blade body, in particular the distal tip end, has a generally parabolic shape defined by a pair of opposing lateral cutting edges and an arcuate distal cutting edge.

In embodiments, the ultrasonic surgical blade and the receptacle are configured such that, upon insertion of the fastening portion into the receptacle, the two proximally oriented prongs are in a flexed state. This creates a force-fit or frictionally engaged connection between the ultrasonic surgical blade and the receptacle, which contributes to a secure fit of the ultrasonic surgical blade in the receptacle and prevents it from falling out of the receptacle. Preferably the two proximally oriented prongs are in a less flexed state compared to the flexed state when the ultrasonic surgical blade and the receptacle are operatively coupled. This ensured good contact between the ultrasonic surgical blade and the receptacle, and thus good transmission of ultrasonic vibrations to the ultrasonic surgical blade, while also reducing the load on the ultrasonic surgical blade.

In the context of the invention, the term "less flexed" may in particular also comprise an unflexed state.

In embodiments, the receptacle has a lumen for supplying an irrigation liquid, wherein the lumen is in liquid communication with the slot when the ultrasonic surgical blade and the receptacle are operatively coupled. The fact that the irrigation liquid or cooling liquid is supplied directly to the proximal slot ensures a particularly clean and efficient supply of liquid to the blade and the surgical site.

In embodiments where the receptacle has a lumen in liquid communication with the slot, the ultrasonic surgical blade and the receptacle form a liquid-tight connection between the lumen and a volume defined between the two proximally oriented prongs and the receptacle. This has the advantage that, unlike in the prior art, where irrigation liquid or cooling liquid often leaks out undesirably to the side or even sprays the surgeon, the irrigation liquid can be supplied to the blade and the surgical site in a controlled manner and without splashing.

In embodiments, the ultrasonic surgical blade comprises at least one internal cooling channel running inside the blade body. The receptacle has a lumen for supplying a cooling liquid, wherein the lumen is in liquid communication with the at least one internal cooling channel when the ultrasonic surgical blade and the receptacle are operatively coupled.

The lumen for supplying an irrigation liquid and the lumen for supplying a cooling liquid may be one and the same lumen or separate, distinct lumens. Accordingly, the irrigation liquid can also be used as the cooling liquid, and vice versa.

In embodiments, the at least one internal cooling channel runs in extension of the slot and inside the blade body. In particular, both the slot and the at least one internal cooling channel run in and along a longitudinal centre line L of the blade body. This ensures uniform cooling of the ultrasonic surgical blade across the longitudinal direction of the ultrasonic surgical blade.

In embodiments, the at least one internal cooling channel discharges at the distal tip end.

In embodiments, the at least one internal cooling channel branches out inside the blade body to form a plurality of cooling channel sections. The cooling channel sections discharge on at least one of the distal tip end, a first lateral side edge of the blade body, and a second lateral side edge of the blade body opposite the first lateral side edge of the blade body. In embodiments where the cooling channel sections discharge on at least one of the lateral side edges of the blade body, it is preferred that the cooling channel sections discharge in the working portion of the blade body. This way, the cooling liquid can also function as irrigation liquid for the working site.

In cases where the liquid used for cooling the ultrasonic surgical blade is either physiologically not well tolerated or should not be released into the environment for some other reason, it is conceivable that the ultrasonic surgical tool, in particular the receptacle of the ultrasonic surgical tool, forms a closed cooling circuit with the ultrasonic surgical blade.

To this end, in embodiments, the receptacle comprises a first lumen for supplying a cooling liquid from a cooling liquid reservoir and a second lumen for returning the cooling liquid to the cooling liquid reservoir. The ultrasonic surgical blade comprises an internal cooling channel running inside the blade body, wherein the internal cooling channel comprises an inlet at a first end of the internal cooling channel and an outlet at a second end opposite the first end of the internal cooling channel. When the ultrasonic surgical blade and the receptacle are operatively coupled, the first lumen and the inlet form a liquid-tight connection between the first lumen and the inlet, and the second lumen and the outlet form a liquid-tight connection between the second lumen and the outlet.

In embodiments of the ultrasonic surgical blade comprising an internal cooling channel, the inlet of the internal cooling channel is located on a first of said proximally oriented prongs and the outlet of the internal cooling channel is located on a second of said proximally oriented prongs. In particular, the inlet and the outlet of the internal cooling channel may be located at the proximal connection end of the blade body and open towards proximally.

A closed cooling circuit between the ultrasonic surgical tool and the ultrasonic surgical blade ensures a particularly clean and safe use of cooling liquid. More specifically, a closed cooling circuit is beneficial because it can prevent or at least reduce the spray of cooling liquid into the surgical site or wound, which can improve the surgeon's view of the surgical site and reduces the risk of introducing contaminants into the wound. Also, the fact that the cooling liquid runs in a closed circuit ensures that the cooling liquid can be recycled and re-used, which saves costs.

While the fastening elements and the retention elements ensure the correct positioning of the ultrasonic surgical blade in the receptacle and prevent the ultrasonic surgical blade from falling out of the receptacle, their engagement with each other may not yet be sufficient to ensure optimal transmission of ultrasonic vibrations.

To this end, in embodiments, the ultrasonic surgical tool further comprises a connecting member. The connecting member is configured to provide or enhance a frictional connection between the ultrasonic surgical blade and the receptacle. The connecting member may in particular be a collet that is threadably engageable with an external thread on the handpiece or receptacle. When rotating the collet relative to the handpiece or receptacle, opposing contact surfaces for the ultrasonic surgical blade, which are part of the handpiece or receptacle, are forced towards one another, clamping the ultrasonic surgical blade between them. Such embodiments have the advantage of providing a particularly secure and tight fit between the ultrasonic surgical blade and the receptacle in the x-, y-, and z-directions, and thus a particularly secure and effective transmission of ultrasonic vibrations.

In embodiments, at least one of the proximally oriented prongs comprises a cutout for reducing a flexural rigidity of the respective proximally oriented prong. This makes it easier to flex the proximally oriented prongs together and facilitates insertion of the ultrasonic surgical blade into the receptacle.

In embodiments, the plane blade body has a length l_{b} of between 15 mm and 40 mm, preferably between 20 mm and 30 mm, measured in the longitudinal direction L between the proximal connection end and the distal tip end. Alternatively or in addition, the plane blade body has a width w_{b} of between 3 mm and 10 mm, preferably between 4 mm and 6 mm, measured transversely to the longitudinal direction L. In particular, the plane blade body has a width w_{b} of between 3 mm and 10 mm, preferably between 4 mm and 6 mm, at the distal tip end.

In embodiments, the working portion of the plane blade body has a length l_{w} of between 20 mm and 30 mm. Alternatively or in addition, the working portion of the plane blade body has a width w_{w} of between 4 mm and 6 mm.

In embodiments, at least one of the first engagement surface and the second engagement surface has a geometric feature complemental to the geometric features of the proximally oriented prongs. Preferably, both the first engagement surface and the second engagement surface have geometric features complemental to the geometric features of the proximally oriented prongs. The geometric features may include a fluted or corrugated surface, which allows the ultrasonic surgical blade to be held particularly securely in the receptacle.

In embodiments, the ultrasonic transducer and the receptacle are formed as a monolithic unit. In other words, the ultrasonic transducer and the receptacle are shaped as a single piece. This type of design is characterized by particularly low ultrasonic losses.

In embodiments, the connecting member, in particular the collet, is provided with opposing wrench flats for tightening the connecting member on the receptacle with a wrench.

In embodiments where the connecting member is a collet, the collet has a screw lock. The or screw lock may in particular be a sealing ring configured to deform upon tightening the collet by hand. This ensures that the collet cannot come loose on its own, i.e. unintentionally, and it ensures that the ultrasonic surgical blade is properly seated in the receptacle and cannot shift in the receptacle, even if the collet is not yet fully tightened on the receptacle or the handpiece.

A tightening torque of the connecting member depends on factors such as the material and the design of the connecting member, as will be understood by the skilled person. In embodiments, the connecting member, in particular the collet, is tightened to a torque of between 2 Nm and 5 Nm, in particular of 2.75 +/- 0.25 Nm. In this range, a balance is achieved between a sufficiently firm connection and thus optimal transmission of ultrasonic vibrations and easy assembly and disassembly by the user.

In embodiments, a resonance frequency of the ultrasonic surgical blade is approximately between 20 kHz and 40 kHz, in particular approximately 26 kHz, when the ultrasonic surgical blade and the receptacle are operatively coupled. At this frequency, the ultrasonic surgical blade achieves particularly good selectivity with regard to hard tissue such as bone, whereas soft tissue is not injured by the ultrasonic surgical blade.

Unless otherwise specified, one, several or all of the embodiments of the ultrasonic surgical tool disclosed herein may be combined with one another.

In another aspect of the invention, the object is solved with an ultrasonic surgical blade, in particular for use in an ultrasonic surgical tool as described herein. The ultrasonic surgical blade comprises a generally plane blade body with a slot, wherein the plane blade body extends, in a longitudinal direction of the ultrasonic surgical blade, between a proximal connection end and a distal tip end. The blade body comprises a proximally located fastening portion and a distally located working portion, wherein the slot extends from the connection end towards the tip end so as to form the fastening portion of the blade body with two proximally oriented prongs.

Such an ultrasonic surgical blade can be manufactured with simple means and at low cost.

In embodiments, at least one, preferably both, of said proximally oriented prongs comprises at least one fastening element engageable with at least one retention element of the receptacle complemental to the fastening element.

The ultrasonic surgical blade can have one or more or all of the features described for the ultrasonic surgical blade in connection with ultrasonic surgical tool. Embodiments of the ultrasonic surgical blade have the advantages already described above in relation of the ultrasonic surgical tool.

### BRIEF DESCRIPTION OF THE DRAWINGS

Embodiments of the present disclosure will now be described by way of example only and with reference to the following accompanying drawings in which the same or corresponding elements are generally labelled with the same reference signs. The drawings show:
- Figure 1: Plan view of an ultrasonic surgical blade according to an embodiment of the invention;
- Figure 2: Plan view of an ultrasonic surgical blade according to another embodiment of the invention;
- Figure 3: Plan view of an ultrasonic surgical blade according to another embodiment of the invention;
- Figure 4: Plan view of an ultrasonic surgical blade according to another embodiment of the invention;
- Figure 5: Plan view of an ultrasonic surgical blade according to another embodiment of the invention;
- Figure 6: Perspective view of a receptacle for receipt of an ultrasonic surgical blade according to an embodiment of the invention;
- Figure 7: Cross-section through a proximally oriented prong of an ultrasonic surgical blade according to an embodiment of the invention, in a receptacle;
- Figure 8: Schematic view of an ultrasonic surgical tool according to an embodiment of the invention;
- Figure 9: Perspective view of an ultrasonic surgical tool according to another embodiment of the invention;
- Figure 10: Schematic view of an ultrasonic surgical tool according to an embodiment of the invention with a closed cooling circuit.

### DESCRIPTION OF THE PREFERRED EMBODIMENTS

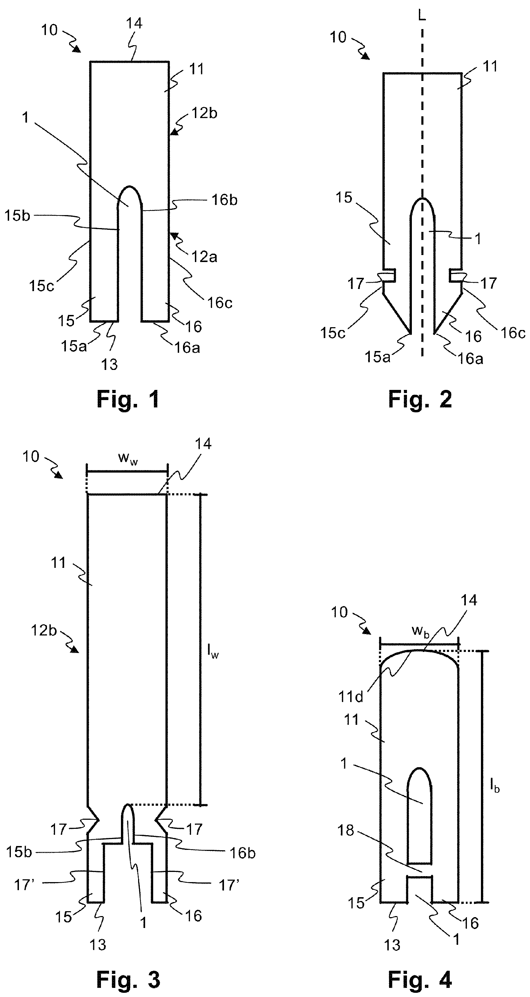

**Figure** 1 shows a plan view of an ultrasonic surgical blade 10 according to an embodiment of the invention. The ultrasonic surgical blade 10 comprises a generally plane blade body 11, i.e. a blade body 11 which is generally plane, with a slot 1. The blade body 11 extends between a proximal connection end 13 and a distal tip end 14. Accordingly, the ultrasonic surgical blade 10 comprises a proximally located fastening portion 12a and a distally located working portion 12b, wherein the slot 1 extends from the connection end 13 towards the tip end 14 so as to form the fastening portion 12a of the blade body 11 with two proximally oriented prongs 15, 16. Each of said proximally oriented prongs 15, 16 has a free end 15a, 16a, an inside edge 15b, 16b facing the slot 1, and an outside edge 15c, 16c facing away from the slot 1.

**Figure** 2 shows a plan view of an ultrasonic surgical blade 10 according to another embodiment of the invention. The generally plane blade body 11 comprises a single slot 1 which runs in and along a longitudinal centre line L of the blade body 11. Accordingly, slot 1 forms and separates two proximally oriented prongs, i.e. a first proximally oriented prong 15 and a second proximally oriented prong 16. In the embodiment shown in Figure 2, each of the proximally oriented prongs 15, 16 features a fastening element 17, which is formed in the shape of a rectangular recess on the respective outside edge 15c, 16c of the proximally oriented prongs 15, 16. However, as will be further described in more detail below, the fastening elements 17 may also have other shapes, i.e. shapes that differ from a rectangular one. For example, one or both of the fastening elements 17 may be a v-shaped, u-shaped, semi-circular or triangular recess in the respective outside edge 15c, 16c of the respective prong 15, 16. The free ends 15a, 16a of the proximally oriented prongs 15, 16 taper in the direction of the respective free ends and towards slot 1 and the longitudinal centre line L. In other words, the free ends 15a, 16a of both proximally oriented prongs 15, 16 taper from the respective outside edges 15c, 16c to the respective inside edges.

**Figure 3** shows a plan view of an ultrasonic surgical blade 10 according to yet another embodiment of the invention. In this embodiment, a generally plane blade body 11 extends between a proximal connection end 13 and a distal tip end 14. The blade body 11 comprises exactly one slot 1 extending from the connection end 14 towards the distal tip end 14. Accordingly, a first proximally oriented prong 15 and a second proximally oriented prong 16 are formed. The first proximally oriented prong 15 comprises, on its outside edge 15c, a first fastening element 17 and, on its inside edge 15b, a second fastening element 17'. Analogously, the second proximally oriented prong 16 comprises a first fastening element 17 on its outside edge 16c and a second fastening element 17' on its inside edge 16b. The first fastening elements 17 are each formed as a v-shaped notch on the respective outside edges 15c, 16c and the second fastening elements 17' are each formed as a rectangular notch on the respective inside edges 15b, 16b. The rectangular notches on the respective inside edges 15b, 16b extend all the way to the connection end 13 of the blade body 11. The blade body 11 comprises a working portion 12b extending between the slot 1 and the distal tip end 14. In the embodiment shown in Figure 3, the working portion 12b of the plane blade body 11 has a length l_{w} of 24 mm, measured in the longitudinal direction of the working portion 12b, and a width w_{w} of 6 mm, measured at the widest point of the working portion 12b.

**Figure 4** shows a plan view of an ultrasonic surgical blade 10 according to yet another embodiment of the invention. The plane blade body 11 has a length l_{b} of 20 mm, measured in the longitudinal direction between the proximal connection end 13 and the distal tip end 14. Moreover, the plane blade body has a width w_{b} of 6 mm, measured transversely to the longitudinal direction at the widest point of the blade body 11. The distal tip end 14 has a generally parabolic shape with an arcuate distal cutting edge 11d. The proximally oriented prongs 15, 16 are connected to each other by a bridging element 18. The bridging element 18 spans the slot 1 and is monolithic with the blade body 11. As can be seen from Figure 4, the ultrasonic surgical blade 10 still features a slot 1 extending from the connection end 13 towards the tip end 14 despite the bridging element 18.

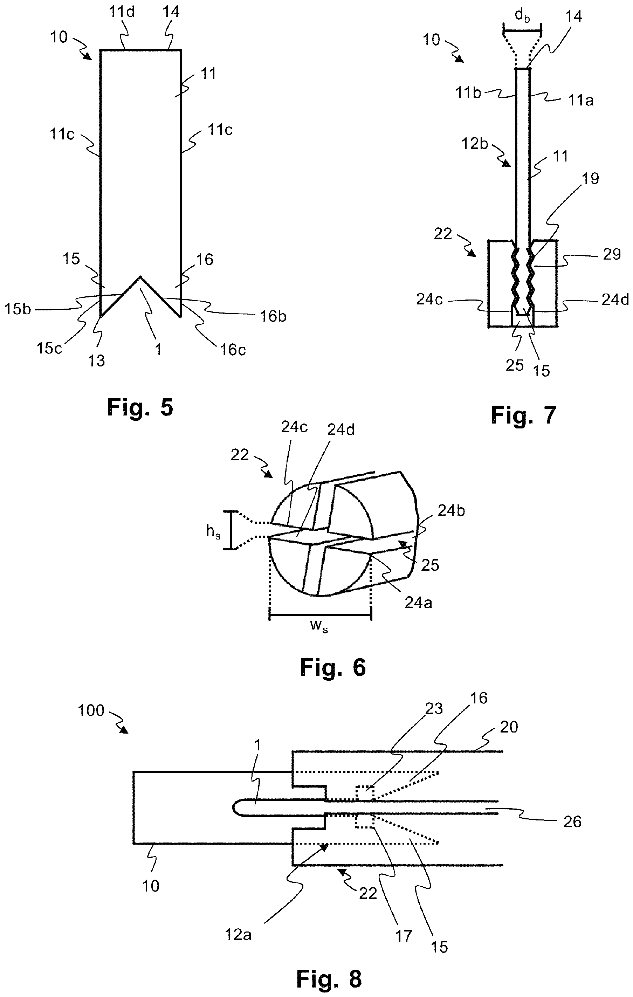

**Figure 5** shows a plan view of an ultrasonic surgical blade according to another embodiment of the invention. Again, the blade body 11 comprises exactly one slot 1 at its proximal connection end 13 giving rise to two proximally oriented prongs 15, 16. In the embodiment shown in Figure 5, the slot 1 is v-shaped, wherein a width of the slot 1 increases in a direction away from the distal tip end 14 and towards the proximal connection end 13. In other words, the two proximally oriented prongs 15, 16 taper in the direction of the connection end 13 from the respective inside edges 15b, 16b to the respective outside edges 15c, 16c. Moreover, the blade body 11 comprises two lateral cutting edges 11c and a distal cutting edge 11d.

**Figure 6** shows a perspective view of a receptacle 22 for receipt of an ultrasonic surgical blade according to an embodiment of the invention. The receptacle 22 comprises a plus-shaped opening with an open front end 24a, a back end 24b opposite the front end 24a, a first engagement surface 24c arranged between the front end 24a and the back end 24b, and a second engagement surface 24d opposite the first engagement surface 24c and arranged between the front end 24a and the back end 24b. The first and second engagement surfaces 24c, 24d define a coupling slot 25 therebetween. In the embodiment shown in Figure 6, the coupling slot 25 has a height hₛ, measured as a distance between the first and the second engagement surfaces 24c, 24d, of approximately 0.8 mm. The width wₛ of the coupling slot 25, measured transversely to its height hₛ, is approximately 4.8 mm. The coupling slot 25 is configured to receive an ultrasonic surgical blade as disclosed herein, wherein at least one of the height hₛ and the width wₛ is dimensioned so as to frictionally engage the ultrasonic surgical blade received in the coupling slot 25. In other words, the height and/or width of the ultrasonic surgical blade and the coupling slot 25 are precisely matched to each other, with the coupling slot 25 having a dimension that is a few tenths of a micrometer smaller than the ultrasonic surgical blade, so that a clamped connection is formed between the receptacle 22 and the ultrasonic surgical blade.

**Figure 7** shows a cross-section through a proximally oriented prong 15 of an ultrasonic surgical blade 10 according to an embodiment of the invention, in a receptacle 22. The blade body 11 comprises a top plane surface 11a, a bottom plane surface 11b, and a blade thickness d_{b}. The blade thickness d_{b} is measured as a distance between the top plane surface 11a and the bottom plane surface 11b, wherein the blade thickness d_{b} refers in particular to the average thickness of the working portion of the blade body 11, i.e. the portion of the blade body 11 that does not comprise the proximal slot. In the embodiment shown in Figure 7, the blade thickness d_{b} is 0.5 mm. The ultrasonic surgical blade 10 is clamped in a coupling slot 25 formed between a first engagement surface 24c and a second engagement surface 24d of the receptacle 22. To this end, the proximally oriented prong 15 comprises, on its top and bottom surfaces 11a, 11b, geometric features 19 in the form of corrugations. Both the first engagement surface 24c and the second engagement surface 24d comprise geometric features 29 complemental to the geometric features 19 of the proximally oriented prong 15.

**Figure 8** shows a schematic view of an ultrasonic surgical tool 100 according to an embodiment of the invention. The ultrasonic surgical tool 100 comprises an ultrasonic surgical blade 10 with a slot 1 at its proximal connection end, and a receptacle 22 surrounded by a handpiece 20. The ultrasonic surgical blade 10 comprises one fastening element 17 on each of its proximally oriented prongs 15, 16. The fastening elements 17 are in the form of rectangular notches located on the respective inside edges of the prongs 15, 16. The fastening portion 12a of the ultrasonic surgical blade 10, which reaches from the start of the slot 1 to the proximal connecting end of the ultrasonic surgical blade 10, is inserted into the receptacle 22 until retention elements 23 complemental to the fastening elements 17 are brought into engagement with each other such that further movement of the ultrasonic surgical blade 10 relative to the receptacle 22 is effectively prohibited. In this state, the ultrasonic surgical blade 10 and the receptacle 22 are operatively coupled, although it is preferred that a further connecting member configured for ensuring a tight fit between the ultrasonic surgical blade 10 and the receptacle 22 is provided, for optimal transmission of ultrasonic vibrations to the ultrasonic surgical blade 10. As will be apparent to the skilled person, it is possible to remove the ultrasonic surgical blade 10 from the receptacle 22 when the fastening elements 17 and the retention elements 23 are separated, e.g. by spreading the two proximally oriented prongs 15, 16, and the ultrasonic surgical blade 10 is pulled forwards out of the receptacle 22 in the direction of the distal tip end. Suitable mechanisms for releasing the fastening elements 17 and the retention elements 23 from their engagement can be provided in the receptacle 22 or the handpiece 20. The handpiece 20 and the receptacle 22 have a lumen 26 that is in liquid communication with the slot 1 and forms a liquid-tight connection with a volume defined between the two proximally oriented prongs 15, 16 and the receptacle 22.

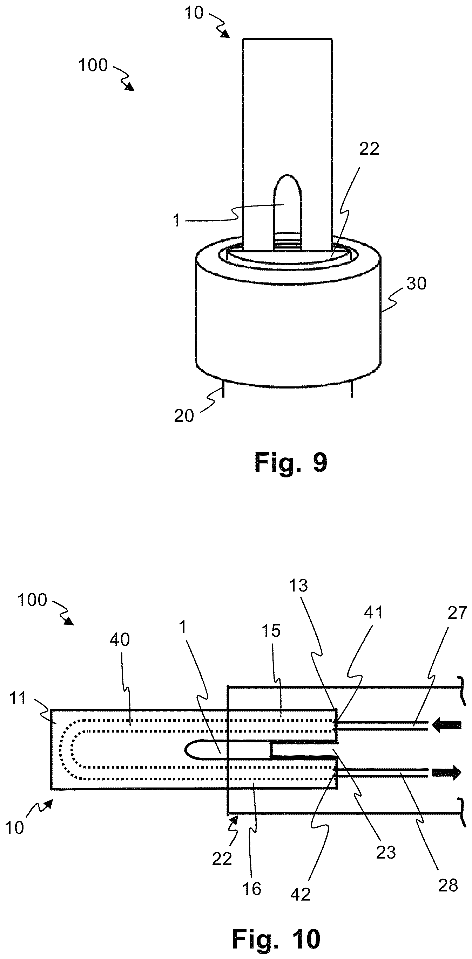

**Figure 9** shows a perspective view of an ultrasonic surgical tool 100 according to another embodiment of the invention. The ultrasonic surgical tool 100, in addition to an ultrasonic surgical blade 10 and a handpiece 20 with a receptacle 22 for said ultrasonic surgical blade 10, comprises a connecting member 30 in the form of a collet. The collet 30 has an internal thread which can be screwed onto a matching external thread on the handpiece 20 such that, when the collet 30 is screwed onto the handpiece 20, it presses on the radially outer surface of the receptacle 22. The receptacle 22 transfers the pressure radially inwardly such that the frictional connection between the receptacle 22 and the ultrasonic surgical blade 10 is enhanced. The slot 1 is dimensioned such that a part of the slot 1 extends beyond the receptacle 22 when the ultrasonic surgical blade 10 abuts the back end of the receptacles opening when the receptacle 22 are operatively coupled. When the collet 30 is tightened, a particularly secure and tight fit is achieved between the ultrasonic surgical blade 10 and the receptacle 22 in the x-, y-, and z-direction such that ultrasonic vibrations can effectively be transferred onto the ultrasonic surgical blade 10.

**Figure 10** shows a schematic view of an ultrasonic surgical tool 100 according to an embodiment of the invention with a closed cooling circuit. The ultrasonic surgical blade 10 and the receptacle 22 are operatively coupled to form a closed cooling circuit. To this end, the receptacle 22 comprises a first lumen 27 for supply a cooling liquid from a cooling liquid reservoir and a second lumen 28 for returning the cooling liquid to the cooling liquid reservoir. The flow direction of the cooling liquid from and to the cooling liquid reservoir, not shown in Figure 10, is indicated in Figure 10 with two arrows. The ultrasonic surgical blade 10 comprises an internal cooling channel 40, which runs inside the blade body 11 of ultrasonic surgical blade 10 and is therefore drawn in Figure 10 with dotted lines. The internal cooling channel 40 comprises an inlet 41 at a first end of the internal cooling channel 40 and an outlet 42 at a second end opposite the first end of the internal cooling channel. In the operatively coupled state shown in Figure 10, the first lumen 27 and the inlet 41 form a liquid-tight connection between the first lumen 27 and the inlet 41 or between the receptacle 22 and the ultrasonic surgical blade 10, respectively. Also, the second lumen 28 and the outlet 42 form a liquid-tight connection between the second lumen 28 and the outlet 42 or between the receptacle 22 and the ultrasonic surgical blade 10, respectively. In the embodiment shown in Figure 10, the inlet 41 of the internal cooling channel 40 is located at the proximal connection end 13 of the blade body 11 and on a first proximally oriented prong 15 formed by slot 1. The outlet 42 of the internal cooling channel 40 is located at the proximal connection end 13 of the blade body 11 and on a second proximally oriented prong 16 formed by slot 1. Both inlet 41 and outlet 42 of open towards proximally. The path of the internal cooling channel 40 shown in Figure 10 is purely exemplary for clarification purposes and may differ, depending on requirements, in its dimensions as well as in its routing inside the blade body 11 from the illustrated embodiment without departing from the inventive concept described herein. In the embodiment shown in Figure 10, the receptacle 22 comprises a retention element 23 in the form of a guide pin comprising a width which essentially corresponds to the width of the slot 1 and thus engages with the two proximally oriented prongs 15, 16 such that the ultrasonic surgical blade 10 is aligned with the receptacle 22 in a defined manner. In other words, in the simplest case, the two proximally oriented prongs 15, 16 themselves may constitute the fastening elements engageable with at least one retention element 23 of the receptacle 22. This way, a frictional or friction-locked connection between the ultrasonic surgical blade 10 and the receptacle 22 is provided, which prevents the ultrasonic surgical blade 10 from falling out of the receptacle 22 after it has been inserted and before it is finally secured with an additional connecting member as disclosed herein, if necessary.

## Claims

1. An ultrasonic surgical tool (100) comprising a handpiece (20) and an ultrasonic surgical blade (10), wherein the handpiece (20) comprises a receptacle (22) for the ultrasonic surgical blade (10), the receptacle (22) being configured for transmitting ultrasonic vibrations from an ultrasonic transducer to the ultrasonic surgical blade (10) when the ultrasonic surgical blade (10) is operatively coupled to the receptacle (22),
wherein the ultrasonic surgical blade (10) comprises a generally plane blade body (11) with a slot (1),
wherein the plane body (11) extends, in a longitudinal direction of the ultrasonic surgical blade (10), between a proximal connection end (13) and a distal tip end (14) and comprises a proximally located fastening portion (12a) and a distally located working portion (12b), and
wherein the slot (1) extends from the connection end (13) towards the tip end (14) so as to form the fastening portion (12a) of the blade body (11) with two proximally oriented prongs (15, 16).

2. The ultrasonic surgical tool (100) according to claim 1, wherein the slot (1) is dimensioned such that a part of the slot (1) extends beyond the receptacle (22) when the ultrasonic surgical blade (10) and the receptacle (22) are operatively coupled.

3. The ultrasonic surgical tool (100) according to claim 1 or 2, wherein the receptacle (22) comprises an opening with an open front end (24a), a back end (24b) opposite the front end (24a), a first engagement surface (24c) arranged between the front end (24a) and the back end (24b), and a second engagement surface (24d) opposite the first engagement surface (24c) and arranged between the front end (24a) and the back end (24b), the first and second engagement surfaces (24c, 24d) defining a coupling slot (25) therebetween for receipt of the ultrasonic surgical blade (10), preferably the back end (24b) defining a locating surface.

4. The ultrasonic surgical tool (100) according to claim 3, wherein the coupling slot (25) has at least one of a height hₛ, measured as a distance between the first and the second engagement surface (24c, 24d), and a width wₛ, measured transversely to the height hₛ, dimensioned so as to frictionally engage the ultrasonic surgical blade (10) received in the coupling slot (25).

5. The ultrasonic surgical tool (100) according to any one of the preceding claims, wherein at least one, preferably both, of said proximally oriented prongs (15, 16) comprises at least one fastening element (17) engageable with at least one retention element (23) of the receptacle (22) complemental to the fastening element (17), wherein the at least one fastening element (17) and the at least one retention element (23) are configured for detachably retaining the ultrasonic surgical blade (10) on the handpiece (20) with the fastening element (17) and the retention element (23) in cooperation.

6. The ultrasonic surgical tool (100) according to any one of the preceding claims, wherein the blade body (11) comprises a top plane surface (11a), a bottom plane surface (11b), and a blade thickness d, measured as a distance between the top plane surface (11a) and the bottom plane surface (11b), wherein the blade thickness d is between 0.1 mm and 1 mm and/or wherein the blade thickness d is essentially constant over at least 80% of the working portion (12b) of the blade body (11).

7. The ultrasonic surgical tool (100) according to any one of the preceding claims, wherein each of said proximally oriented prongs (15, 16) has a free end (15a, 16a), an inside edge (15b, 16b) facing the slot (1), an outside edge (15c, 16c) facing away from the slot (1), a top surface (15d, 16d) and a bottom surface (15e, 16e) opposite the top surface (15d, 16d), wherein said at least one fastening element (17) is selected from the group consisting of
- a protrusion extending laterally outwardly on its outside edge (15c, 16c);
- a notch on its outside edge (15c, 16c);
- a protrusion extending laterally inwardly on its inside edge (15b, 16b);
- a notch on its inside edge (15b, 16b);
- a free end (15a, 16a) tapering in the direction of the free end (15a, 16a), in particular a free end (15a, 16a) tapering in the direction of the free end (15a, 16a) and towards the slot (1);
- a taper of at least one of said proximally oriented prongs (15, 16) away from the free end (15a, 16a) and towards the distal tip end (14);
- a geometric feature (19), in particular a corrugation, for increasing friction of the fastening portion (12a) with the receptacle (22).

8. The ultrasonic surgical tool (100) according to any one of the preceding claims, wherein the proximally oriented prongs (15, 16) are connected to each other by at least one bridging element (18), the bridging element (18) spanning the slot (1) and being monolithic with the blade body (11).

9. The ultrasonic surgical tool (100) according to any one of the preceding claims, wherein the working portion (12b) of the blade body (11) comprises at least one cutting edge.

10. The ultrasonic surgical tool (100) according to claim 9, wherein the blade body (11) has a generally rectangular shape defined by a pair of opposing lateral cutting edges (11 c) and a straight distal cutting edge (11 d).

11. The ultrasonic surgical tool (100) according to any one of the preceding claims, wherein the ultrasonic surgical blade (10) and the receptacle (22) are configured such that, upon insertion of the fastening portion (12a) into the receptacle (22), the two proximally oriented prongs (15, 16) are in a flexed state; and preferably wherein the two proximally oriented prongs (15, 16) are in a less flexed state compared to the flexed state when the ultrasonic surgical blade (10) and the receptacle (22) are operatively coupled.

12. The ultrasonic surgical tool (100) according to any one of the preceding claims, the receptacle (22) having a lumen (26) for supplying an irrigation liquid, the lumen (26) being in liquid communication with the slot (1) when the ultrasonic surgical blade (10) and the receptacle (22) are operatively coupled.

13. The ultrasonic surgical tool (100) according to claim 12, wherein the ultrasonic surgical blade (10) and the receptacle (22) form a liquid-tight connection between the lumen (26) and a volume defined between the two proximally oriented prongs (15, 16) and the receptacle (22).

14. The ultrasonic surgical tool (100) according to any one of the preceding claims, further comprising a connecting member (30), in particular a collet being threadably engageable with an external thread on the handpiece (20) or the receptacle (22), wherein the connecting member (30) is configured to provide or enhance a frictional connection between the ultrasonic surgical blade (10) and the receptacle (22).

15. An ultrasonic surgical blade (10), in particular for use in an ultrasonic surgical tool (100) according to any one of the preceding claims, the ultrasonic surgical blade (10) comprising a generally plane blade body (11) with a slot (1), wherein the plane body (11) extends, in a longitudinal direction of the ultrasonic surgical blade (10), between a proximal connection end (13) and a distal tip end (14) and comprises a proximally located fastening portion (12a) and a distally located working portion (12b), and wherein the slot (1) extends from the connection end (13) towards the tip end (14) so as to form the fastening portion (12a) of the blade body (11) with two proximally oriented prongs (15, 16).
